# EUROPEAN PATENT SPECIFICATION

(11) **EP 4 297 807 B1**
(45) Date of publication and mention of the grant of the patent: **24.12.2025**
(21) Application number: 22709805.0
(22) Date of filing: 24.02.2022
(51) Int. Cl.: A61L 27/20, A61L 27/22, A61L 27/36, A61L 27/38, A61L 27/54, B33Y 10/00, B33Y 70/00, B33Y 80/00

(54) **SILK FIBROIN AND RELATED USE FOR 3D BIOPRINTING**
SEIDENFIBROIN UND VERWANDTE VERWENDUNG FÜR 3D-BIODRUCK
FIBROÏNE DE SOIE ET UTILISATION ASSOCIÉE POUR LA BIO-IMPRESSION 3D

(30) Priority: 25.02.2021 IT 202100004412
(43) Date of publication of application: 03.01.2024
(73) Proprietor: Università Degli Studi di Pavia, 27100 Pavia (PV) (IT); CELLINK Bioprinting AB, 412 77 Göteborg (SE)
(72) Inventor: BALDUINI, Alessandra, 27100 Pavia (IT); DI BUDUO, Christian Andrea, 27013 Chignolo Po (PV) (IT); LAURENT, Pierre-Alexandre, 69130 Ecully, Lione (FR); GATENHOLM, Erik, 41346 Gothenburg (SE); MARTINEZ, Hector, 41346 Gothenburg (SE); REDWAN-NAMRO, Itedale, 41346 Gothenburg (SE); KUZMENKO, Volodymyr, 41346 Gothenburg (SE)
(74) Representative: Bird & Bird Società tra Avvocati S.r.l.
(86) International application number: PCT/IB2022/051635
(87) International publication number: WO 2022/180565

(56) References cited:
- CN-A- 107 998 449
- US-A1- 2021 001 009
- ZOU QIANG ET AL: "Biofabrication of valentine-shaped heart with a composite hydrogel and sacrificial material", MATERIALS SCIENCE AND ENGINEERING C, ELSEVIER SCIENCE S.A, CH, vol. 108, 10 November 2019 (2019-11-10), XP086042454, ISSN: 0928-4931, [retrieved on 20191110], DOI: 10.1016/J.MSEC.2019.110205

## Description

The present invention relates to silk fibroin bioinks specifically formulated for use in 3D bioprinting and the production of *ex-vivo* models capable of supporting hematopoiesis and the production of platelets and blood cells.

3D bioprinting is the process of generating 3D spatially-controlled organ and tissue models, wherein the functionality and vitality of the cells are preserved within the printed model (1).

The use of 3D bioprinting for the manufacturing of biological constructs typically involves the delivery of cells within a biocompatible scaffold using a layer-by-layer ink-extrusion approach for generating three-dimensional structures similar to native tissues (1). As every tissue in the body is naturally compartmentalized by different types of cells, many technologies for printing these cells vary in their capacity of ensuring cell vitality and functionality during the manufacturing process. Some of the methods used for the 3D bioprinting of cells are photolithography, magnetic bioprinting, stereolithography and extrusion (2-4).

Bioinks play a fundamental role in bioprinting as they can be adapted for printing complex tissue models, providing the final scaffold with mechanical and physicochemical characteristics (elasticity, hardness, shape) and sufficient cell vitality for preserving its functionality and obtaining a physiological response. (5).

The inks developed for 3D printing are primarily composed of thermoplastics, silicone, collagen, and gelatin or alginate (6). The patent document CN 107 998 449 A, for example, discloses a bioink formulation comprising: alginic acid, gelatin, silk fibroin added as a solution with a polyol, such as glycerin, propylene glycol or xylitol.

The advantage of using silk fibroin in bioinks is that the formulation is transparent, versatile, stable in water and does not require any processing or vulcanization, such as chemical modifications or high-temperature treatments, which would limit its versatility (7).

The great interest and therapeutic potential of hematopoietic stem cells has seen the development of an impressive number of techniques aimed at their isolation and expansion, in order to cultivate hematopoietic stem cells under experimental conditions that reproduce what takes place in the bone marrow, the hematopoietic tissue of adults contained within the bone trabeculae, difficult to reproduce in plates (8-10).

Bone marrow stromal cells promote and regulate the renewal, differentiation and proliferation of hematopoietic stem cells and hematopoietic progenitors by secreting cytokines and components of the extracellular matrix in the marrow micro-environment (11-13).

Stroma-mediated hematopoiesis was studied *in vitro* using the Dexter flask culture system (14). In this system, stromal cells form an adherent layer on which hematopoietic stem cells adhere weakly. Hematopoiesis in the Dexter culture system is almost exclusively limited to the granulocyte line, so other lines cannot expand. Two-dimensional culture systems are capable of expanding hematopoietic progenitors, even in the absence of stroma, but in the presence of large quantities of cytokines, in concentrations normally higher than physiological concentrations (15). This allows the expansion and differentiation of hematopoietic stem cells, but it is an expensive procedure that does not transmit the complexity of the biophysical signals of the 3D medullary environment to the cells *in vivo.*

In this scenario, 3D cell systems represent a valid and promising alternative approach to 2D systems, they are therefore the link between traditional cell culture and *in vivo* models.

The authors of the present invention have now identified a silk fibroin-based bioink formulation having the desired morphological and rheological properties for the 3D bioprinting of a bone marrow tissue that supports hematopoiesis and *ex vivo* platelet production, or the creation of an *in vivo* transplantable system. This 3D bone-marrow tissue can be validly used in medical devices for the targeted release of drugs, for the preparation of preclinical drug assays, as a surgical implant for regenerative medicine or for use in transfusion medicine.

The silk fibroin bioink formulations according to the invention were tested by culturing different cells embedded within the different printed scaffolds, such as human leukemia cell lines, hematopoietic stem cells, genetically modified hematopoietic stem cells, mature blood cells and induced pluripotent stem cells, thus showing a wide versatility of use. The silk fibroin-based bioink formulations according to the invention can be stored for up to two months and, following printing, can keep the different cell types incorporated in the printed tissues and the activity of the molecules included therein alive in the long term (i.e., enzymes, substrates and intermediates of enzymatic reactions, extracellular matrices, drugs, cytokines, chemokines, growth factors, hormones, proteins and glycoproteins).

The present invention therefore relates to a bioink formulation comprising:

| | |
|---|---|
| - silk fibroin | 0.5% -35% weight/volume (w/v) |
| - gelatin or derivatives thereof | 5-20% w/v |
| - alginic acid or derivatives thereof | 0.5-2% w/v |
| - glucose | 3-4% w/v |
| - controlled density solution | 1-3% w/v |
| - buffer | 4-6% w/v |
| - saline buffer solution | up to 100% |

wherein the gelatin and alginic acid or their derivatives are present in a reciprocal ratio ranging from about 10:1 to about 20:1;
wherein the silk fibroin and the gelatin are present in a reciprocal ratio ranging from about 1: 5 to about 1: 0.5; suitable for extrusion with hematopoietic cells.

According to a particular embodiment, the silk fibroin-based bioink formulation further comprises albumin at 0.1% w/v.

In a preferred embodiment, the silk fibroin-based bioink formulation according to the invention further comprises a cell component selected from the group consisting of progenitor cells of the myeloid or lymphoid lineage, hematopoietic stem cells or differentiated cells of the hematopoietic lineage, hematopoietic stem cells or differentiated cells of the hematopoietic lineage genetically modified (e.g. transfection with plasmids, lentivirus treatment, CRISPR/Cas9 system) in a concentration equal to 0.5-4x10⁶ cells/ml, preferably 1x10⁶ cells/ml.

The cell component can be part of the initial bioink formulation or it can also be added after extrusion.

In a preferred embodiment, the saline buffer solution contains:
- 2 mM NaCl
- 6 mM KCl
- 1 mM MgCl₂
- 10 mM HEPES.

According to a preferred embodiment of the invention, the silk fibroin-based bioink formulation is sterile. The sterility of the formulation or its individual components can be advantageously achieved by means of UV-ray sterilization treatment.

The temperature of the silk fibroin bioink formulation is 37°C when the cells are to be added.

The above-mentioned stem or differentiated cells of the hematopoietic lineage in the silk fibroin-based bioink formulation of the invention are preferably mammalian cells, preferably of human origin (not embryonic). Stem or differentiated cells of the hematopoietic lineage can be printed with supporting stromal cells such as fibroblasts and mesenchymal stem cells or with endothelial cells.

Stem or differentiated cells of the hematopoietic lineage can derive directly from primary cell cultures or from cell lines, preferably from multipotent or pluripotent stem cells.

More specifically, they can be progenitor cells (such as, for example, progenitors of the myeloid or lymphoid lineage, megakaryocytes), hematopoietic stem cells (totipotent or pluripotent) or differentiated cells of the myeloid or lymphoid lineage. The differentiated cells of the myeloid lineage derive from a myeloid progenitor and are selected from the group that comprises monocytes, macrophages, granulocytes, neutrophils, basophils, eosinophils, red blood cells, megakaryocytes, platelets, dendritic cells. The differentiated cells of the lymphoid lineage derive from a progenitor and are T lymphocytes, B lymphocytes and NK cells. According to a particular embodiment of the 3D printed scaffold according to the invention, the cells are human hematopoietic stem and CD34⁺ progenitor cells.

The stem or differentiated cells of the hematopoietic lineage and supporting stromal cells can be genetically modified (e.g., plasmid transfection, lentivirus treatment, CRISP/Cas9 system) before being included in the bioink.

In a preferred embodiment of the silk fibroin-based bioink formulation according to the invention, the gelatin and alginic acid or their derivatives are present in a reciprocal ratio of about 15 1.

Again in accordance with a preferred embodiment of the silk fibroin-based bioink formulation according to the invention, the fibroin and gelatin or its derivatives are present in a reciprocal ratio equal to about 1:5 or about 1:2 or 1: *0.5.*

Optionally, the silk fibroin is dissolved in a solution comprising one or more ion sources selected from the group consisting of alkaline or alkaline earth metal chlorides, such as for example MgCl₂, CaCl₂, NaCl, KCl.

The silk fibroin can be of a natural origin, such as, for example, fibroin produced by arthropods such as silkworms (e.g., *Bombyx mori, Anthereae pernyi*) or spiders (e.g., *Nephila clavipes, Araneus diadematus*). Alternatively the silk fibroin can be of a recombinant origin, such as for example the fibroin produced by engineered systems (e.g., bacteria). In a preferred embodiment, the bioink formulation uses fibroin obtained from silkworm cocoons of the *Bombyxa mori* species after boiling for 20 minutes in a 0.02 M Na₂CO₃ solution. Alternatively, the fibroin can be reconstituted from a lyophilized formulation.

The gelatin used in the silk fibroin-based bioink formulation according to the invention can be type A (acid hydrolysis) or type B (alkaline hydrolysis). Preferably the gelatin is of type A.

According to a preferred embodiment of the formulation according to the invention, the gelatin derivatives are selected from the group consisting of gelatin conjugated with chitosan, gelatin-poly(DL-lactide), gelatin modified with PEG, gelatin thiolates, DNA-gelatin nanospheres, gelatin nanoparticles, gelatin-coated fluorescent maghemite nanoparticles, gelatin-coated fluorescent polymethacrylic acid nanoparticles (FPMAAG), supramolecular gelatin nanoparticles coated with quantum dots, gelatin nanoparticles coated with iron oxide, gelatin methacrylate, xanthan gum human gelatin, recombinant gelatin, gelatin marked with fluorescent molecules.

According to a preferred embodiment of the formulation, the alginic acid derivatives are salts with alkaline and alkaline earth metals selected from the group consisting of sodium alginate, calcium alginate, magnesium alginate, potassium alginate. Alternatively, methacrylate alginate can be used for the production of photopolymerizable bioinks in the presence of soluble photoinitiators. Disclosed are glucose analogues selected from the group consisting of disaccharides, stereoisomers, isomers, epimers, alditols or acids of glucose, precursors or products deriving from the glucose metabolism. Glucose analogues refer to metabolites that can be transported within the cell, become part of the glycolysis cycle and metabolized.

Other sugars, such as xylose, do not form part of the present invention as they are not capable of entering the cell via glucose transporters and cannot enter the glycolysis cycle. The cells are therefore not capable of metabolizing them.

By way of example, the glucose analogues disclosed can be selected from the group consisting of: D-glucose, L-glucose, lactose, sucrose, trehalose, cellobiose, melibiose, maltose (disaccharides), mannitol, galactose, mannose, fructose (epimers), allose, altrose (stereoisomers), gluconic acid, glucuronic acid (acids), arabinose, glycerol, pyruvate, glucose 1-phosphate, glucose 6-phosphate, lactate, malate, phosphoglycerate, succinate (precursors or derivatives of the glucose metabolism), sorbitol (alditol). The glucose can also be replaced by fluorescent analogues that allow the glucose transportation to be monitored (e.g. 2-(N-(7-nitrobenz-2-oxa-1,3-diazol-4-yl)amino)-2-deoxyglucose) or analogues that inhibit the glycolytic metabolism (e.g. 2-deoxy-d-glucose).

In further preferred embodiments of the invention the controlled density solution is a controlled density solution selected from the group as defined in the claims. Also disclosed are Percoll^{®} (colloidal silica particles of 15-30 nm in diameter (23% w/w in water) coated with polyvinylpyrrolidone), Ficoll^{®} (sucrose and epichlorohydrin copolymer), colloidal silica, copolymers (e.g. sucrose and epichlorohydrin), hydrophilic polysaccharides (e.g. sucrose), high-molecular-weight synthetic polymers (e.g. Polysucrose^{®}).

The choice of the controlled density solution depends on the degree of density to be attributed to the ink for optimizing printing, favouring the distribution of cells in the suspension and preserving their vitality during the printing process.

Further preferred embodiments of the bioink formulation according to the invention provide that the buffer be an organic or mineral buffer selected from the group consisting of: MES, ADA, ACES, PIPES, MOPSO, Bis-6Tris Propane, BES, MOPS, TES, HEPES, DIPSO, MOBS, TAPSO, HEPPSO, POPSO, EPPS (HEPPS), Tricine, Gly-Gly, Bicine, HEPBS, TAPS, AMPD, TABS, AMPSO, CHES, CAPSO, AMP, CAPS and CABS. The buffer is preferably HEPES (4-2-hydroxyethyl-1-piperazinyl-ethanesulfonic acid) which has a strong buffering power.

Based on the culture conditions required for ensuring vitality and functionality of the cell type being printed, the ideal buffer system is selected that allows the desired pH to be maintained during the 3D printing, the subsequent culture of the printed tissue, and in any type of manipulation and/or analysis of the printed tissue outside the incubator and/or in an uncontrolled CO₂ atmosphere.

The presence of the buffer, for example, is an advantage during the 3D printing process (most commercial printers do not have CO₂-controlled systems). In this way, it is possible to keep the cells viable as they are maintained at a physiological pH even during the 3D printing.

A further advantage is when viewing for a long time using microscopy systems that are not CO₂ controlled. The bioink formulation according to the invention always maintains a physiological pH.

Without a buffer, the bioink formulation can lose its physiological pH and this results in a reduction in cell vitality/functionality.

In a preferred embodiment, the bioink formulation according to the invention has the following composition:

| | |
|---|---|
| - silk fibroin | 8% w/v |
| - gelatin | 15% w/v |
| - alginic acid | 1% w/v |
| - glucose | 3.5% w/v |
| - Percoll^{®} | 2.5% w/v |
| - HEPES | 5% w/v |
| - saline buffer solution | up to 100% |

In a particularly preferred embodiment, the silk fibroin-based bioink formulation comprises albumin 0.1% w/v.

According to a further embodiment of the present invention, the silk fibroin-based bioink formulation can further comprise a fluorescent marker, a contrast agent, an enzyme and/or enzymatic reaction intermediates, a luminescent substance, a chemiluminescent substance, a radio-opaque agent, a radioactive element or a conjugated or unconjugated antibody.

The present invention also relates to the use of the silk fibroin-based bioink formulation according to the invention for the production by 3D bioprinting of scaffolds comprising stem or differentiated cells of the hematopoietic lineage capable of supporting hematopoiesis and the production of platelets and blood cells. The stem or differentiated cells of the hematopoietic lineage capable of supporting hematopoiesis and the production of platelets and blood cells can be genetically modified (e.g., transfection with plasmids, treatment with lentivirus, CRISPR/Cas9 system) before being included in the bioink.

According to a further embodiment of the present invention, the stem or differentiated cells of the hematopoietic lineage can be marked with fluorescent antibodies, conjugated or unconjugated, for the visualization of membrane antigens and/or line-specific receptors (e.g., anti-CD3, anti-CD4, anti-CD8, anti-CD34, anti-CD41, anti-CD42a, anti-CD42b, anti-CD61, anti-CD117, anti-CD235, anti-cMpl) molecular markers (e.g., GFP, TOMATO), fluorescent cytoplasmic tracers (e.g., calcein, carboxyfluorescein succinimidyl ester (CFSE), CellTrac Far Red, CellTracker Deep Red), membrane tracers (e.g. biotinylated or fluorescent lipids and/or phospholipids, 4,4-Difluoro-1,3,5,7,8-Pentamethyl-4-Bora-3°,4°-Diaza-s-Indacene, Annexin V, fluorescent cholesterol markers), organelles (e.g. fluorescent tracers of mitochondria, endoplasmic reticulum, lysososm, Golgi apparatus, granules) and/or nucleus (e.g. propidium iodide, 4',6-diamidino-2-phenylindole, Hoechst), tracers of intracellular calcium movements (e.g. Fura-2 AM, Fluo-3 AM, Fluo-4 AM, Rhod-2 AM, Calcium GreenTM-AM). The marking can be effected before the 3D bioprinting or following 3D culture inside the printed scaffold.

The silk fibroin-based bioink formulation according to the invention must be sterile and at a temperature of 37°C when used in the 3D bioprinting of scaffolds that include stem or differentiated cells of the hematopoietic lineage.

The present invention also relates to a method for the production of a 3D scaffold comprising the following steps:
a) bioprinting of the silk fibroin-based bioink formulation according to the invention through a nozzle having a caliber ranging from 18G to 27G at an extrusion pressure within the range of 5-200 kPa and at a printing rate within the range of 5-1,000 mm/min on a substrate to obtain a mono or multilayer 3D scaffold;
b) bimmersion of the 3D scaffold obtained in step a) in a solution that favours crosslinking of the alginate or exposure to ultraviolet light that favours photopolymerization;
c) addition of the cell culture medium to the scaffold.

The nozzle preferably has a caliber of 18G, 20G or 22G. The extrusion pressure is preferably 5 kPa, 8 kPa, 10 kPa, 12 kPa, 16 kPa or 20 kPa. The printing rate is again preferably within the range of 300-1,000mm/min.

The silk fibroin-based bioink formulation is sterile and at a temperature of 37°C when used in 3D bioprinting.

The scaffold can have one or more layers, preferably more than 3 layers, even more preferably more than 10 layers. The thickness of each single layer can vary from about 0.5 µm to about 800 µm, preferably from about 1 µm to about 400 µm.

The substrate on which the deposition is effected can be any surface selected from the following non-limiting examples: plastic, glass, glass for microscopy (e.g. 0.13-0.17 mm), paper, polymers, natural or synthetic fabrics, metals, silicone, films, semiconductor materials, substrates based on biopolymers (agarose, collagen, gelatin), resins. The substrate can also be a biological substrate such as cells, tissues or organs. The substrate is preferably a cell culture plate or a cell culture plate provided with a glass bottom for microscopy. According to a further preferred embodiment, the substrate can be a cell culture plate made of biocompatible material and with a glass bottom for microscopy (e.g., 0.13-0.17 mm), having the same shape as the printed scaffold, with a reservoir for the deposition of liquids ensuring chamber humidification during microscopic viewing.

According to a further embodiment, the customized culture plate can provide for the integration of control systems of the temperature and CO₂.

The crosslinking solution of step b) is preferably a CaCl₂ solution. The crosslinking solution again preferably contains 0.05-0.1 M CaCl₂ dissolved in a buffer solution containing 2 mM NaCl, 6 mM KCl, 1 mM MgCl₂, 5 mM glucose, 10 mM HEPES. The scaffold is immersed in the crosslinking solution for a minimum of 10 minutes. The scaffold can be subsequently kept in a culture medium according to the experimental requirements.

The present invention further relates to a three-dimensional scaffold obtained by extruding the silk fibroin-based ink formulation described above. Said scaffold can further comprise stem or differentiated cells of the hematopoietic lineage which can be included in the initial formulation or added after the bioprinting process..

According to a further preferred embodiment of the present invention, the scaffold can further comprise one or more molecules selected from the group consisting of:
- components of the extracellular matrix such as, for example, proteoglycans, hyaluronic acid, collagens, elastin, fibronectin, fibrin, fibrinogen, laminins, thrombospondin;
- growth factors, cytokines and chemokines, preferably hematopoietic, such as, for example, interleukins, CSF-1, G-CSF, GM-CSF, SCF, FLT3-L, TPO, EPO, SDF-1α; TGF-β1, TNFα, VEGF, FGF, Notch ligands, WNT, Angiopoietin-1, BMP, IGF-2 and fragments or variants thereof;
- plasma proteins or glycoproteins, such as albumin, globulins, transferrin;
- antigens, conjugated or unconjugated antibodies, or their fragments such as CDRs or epitopes;
- hormones, such as insulin, glucagon, triiodothyronine, thyroxine, or their antagonists;
- drugs or prodrugs, such as TPO mimetics, TPO receptor agonists, tyrosine kinase receptor agonists, tyrosine kinase receptor inhibitors, Rho kinase inhibitors (ROCK), kinase inhibitors (e.g. JAK inhibitors), receptor antagonists of aryl hydrocarbons, chemotherapeutic agents;
- nucleic acids, such as DNA, RNA, siRNA, RNAi and microRNA, plasmids, lentiviruses, CRISPR (Clustered Regularly Interspaced Short Palindromic Repeats);
- enzymes, such as horseradish peroxidase, and/or their substrates or intermediates of enzymatic reactions;
- toxins.

The present invention further relates to the use of the scaffold described above as an *ex vivo* model for supporting hematopoiesis or for the production of platelets and blood cells through the use of growth factors or mediums that maintain the staminality or promote cell differentiation.

The invention also relates to the scaffold described above for use as a surgical implant for supporting hematopoiesis or the production of platelets and blood cells *in vivo.* In a preferred embodiment of the invention said implant further comprises one or more drugs, hormones, growth or differentiation factors that can be released *in situ.*

The present invention further relates to a solution for the dissolution of the scaffold as defined in the claims and the release of the printed cells, keeping them vital and functional.

In a preferred composition embodiment, said solution consists of sodium citrate, collagenase and alginate lyase and a source of glucose.

In a preferred embodiment the formulation of the bioink dissolution solution comprises:

| | |
|---|---|
| - sodium citrate | 15 mM |
| - collagenase | 10 U/mL |
| - alginate lyase | 5 U/mL |
| - glucose | 5 mM |
| - saline buffer solution | up to 100% |

The melting temperature of the silk fibroin-based bioink is preferably 37°C for 10-20 minutes.

Also disclosed is the use of cells released from the scaffold for carrying out morphological, functional, biochemical and/or molecular analyses which can comprise, by way of example, one or more of the following methods:
- collection of proteins for proteomics, immunoassays, electrophoresis and/or Western blotting assays;
- collection of nucleic acids for genomic characterization, evaluation of the cell karyotype, gene expression analysis via polymerase chain reaction in all of its variants and/or gene sequencing;
- immuno-characterization of the released cells;
- imaging;
- enzymatic or colorimetric assays;
- flow cytometric analysis and/or selection of specific cell populations;
- use of cells for functional assays.

The present invention will now be described for illustrative but non-limiting purposes, according to a preferred embodiment with particular reference to the attached figures, in which:

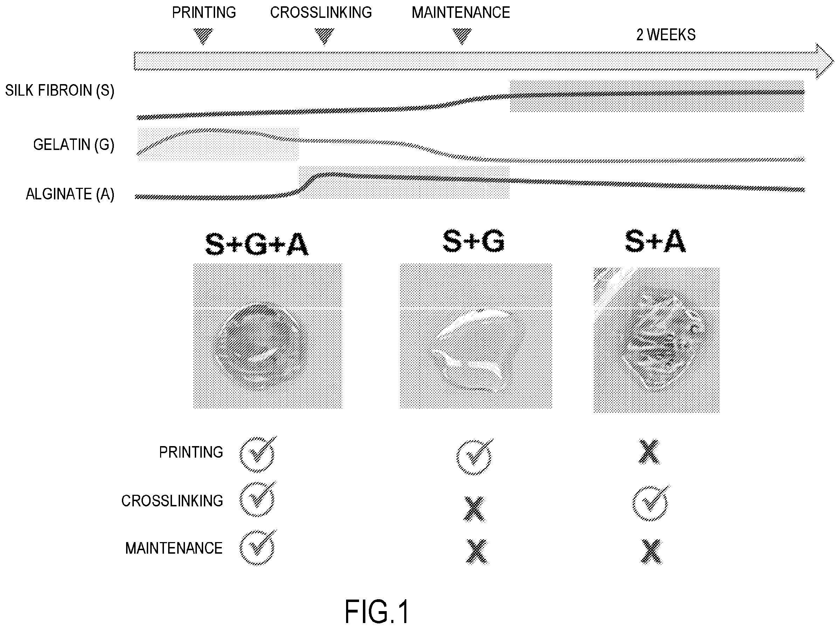

- Figure 1 shows the comparison between the characteristics (Printing, Crosslinking and Maintenance) of the formulation B (S+G+A) tested in comparison with the variants S+G or S+A, and reveals the clearly superior profile of the formulation comprising silk fibroin, gelatin and alginate (S+G+A).

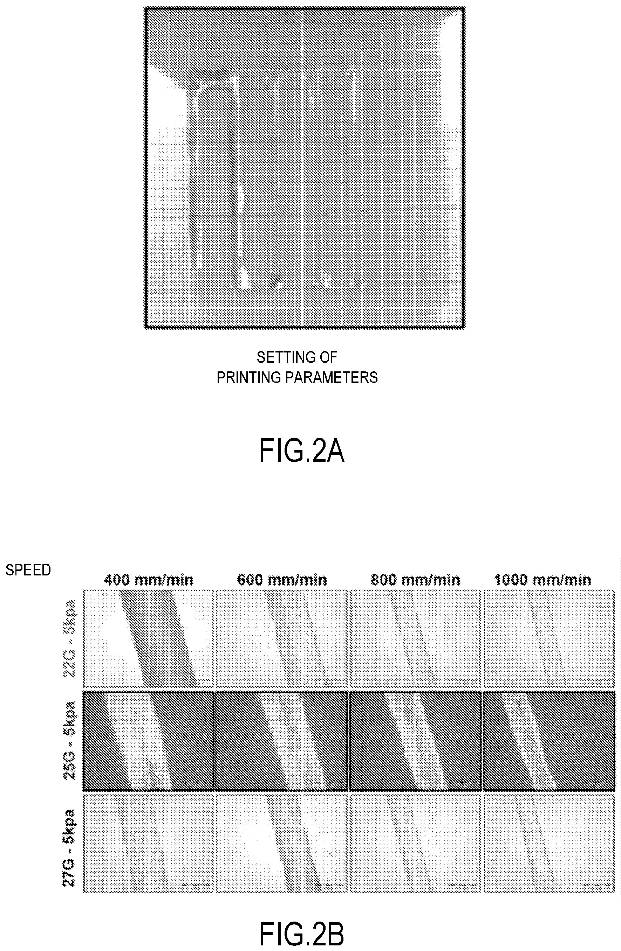

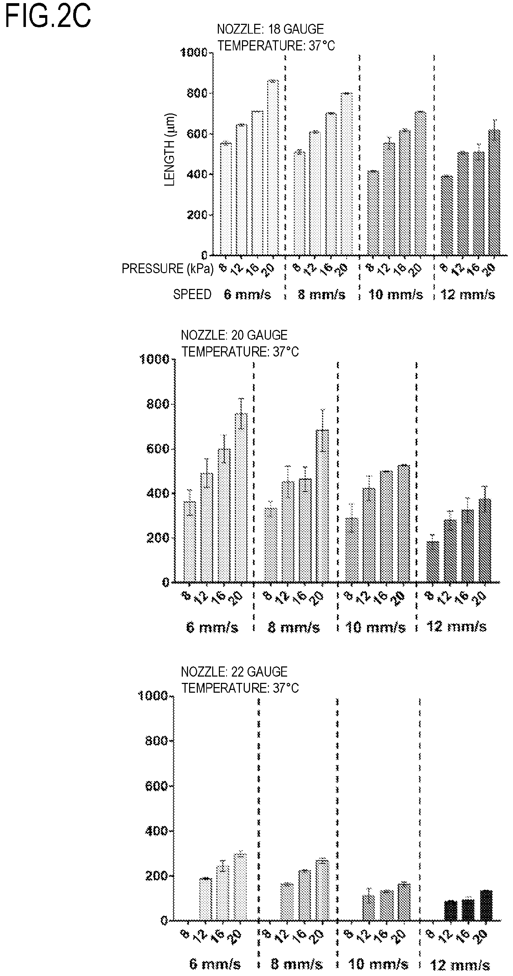

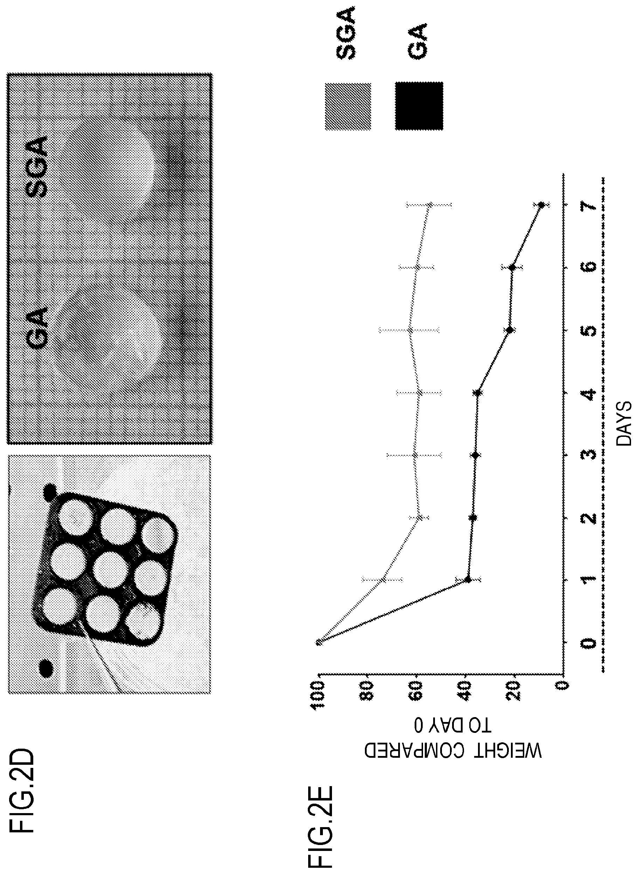

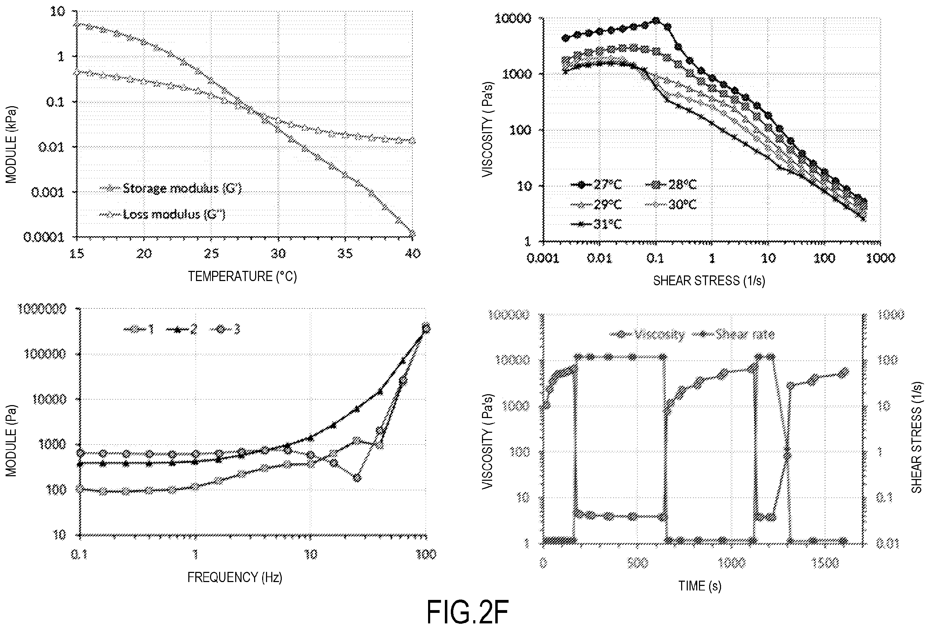

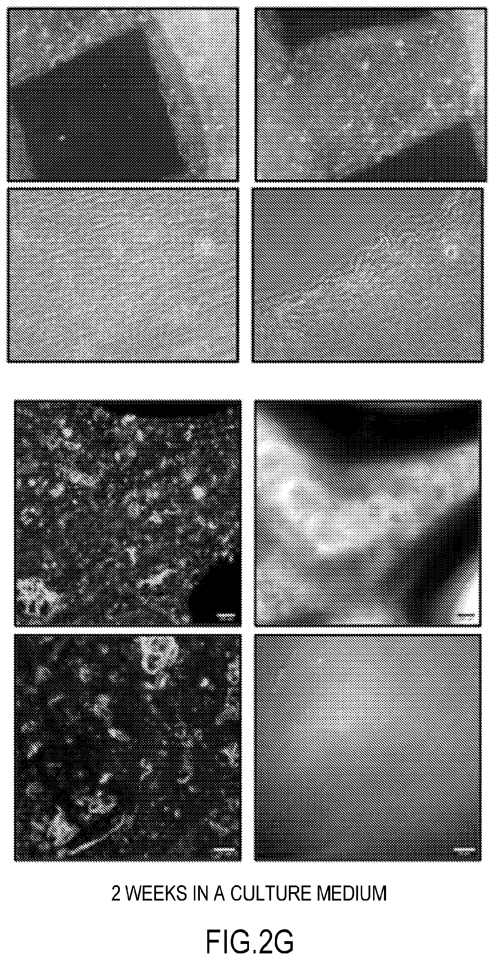

- Figure 2 illustrates the graphs relating to the parameters of the bioprinting process (panel A) with the silk fibroin ink (SGA) of the invention relating to the extrusion pressure and ink deposition rate (panel B); size of the nozzle, extrusion pressure and ink deposition rate (panel C); impact of the presence of fibroin in the sample (SGA) vs sample (GA) (panels D-E); viscosity of the ink SGA (panel F); analysis of the fibroin fibril formation by optical or fluorescence microscopy (panel G).

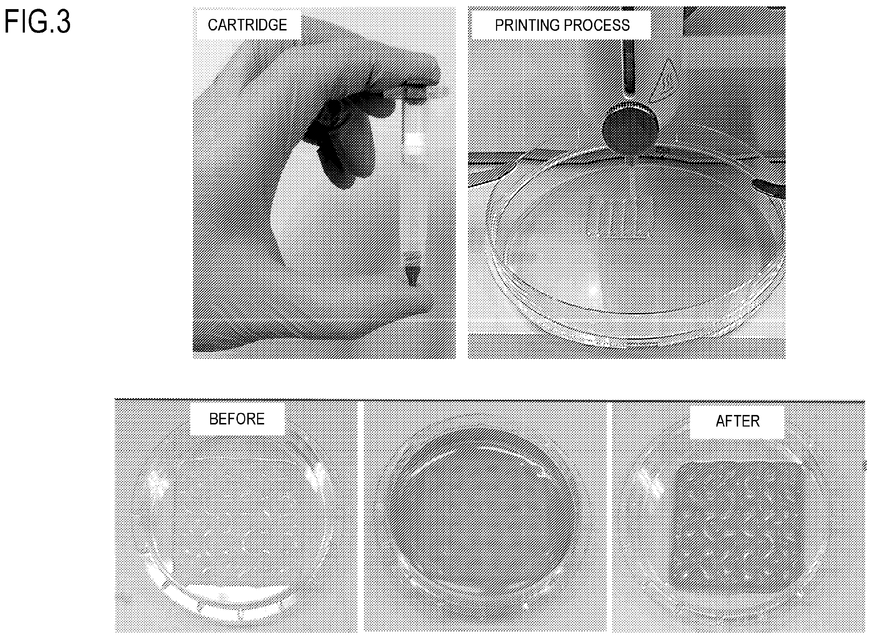

- Figure 3 shows images of the bioprinting process (panels A-B) with the bioink formulation according to the invention and the diffusion capacity of biocompatible dyes in the culture medium (panel C).

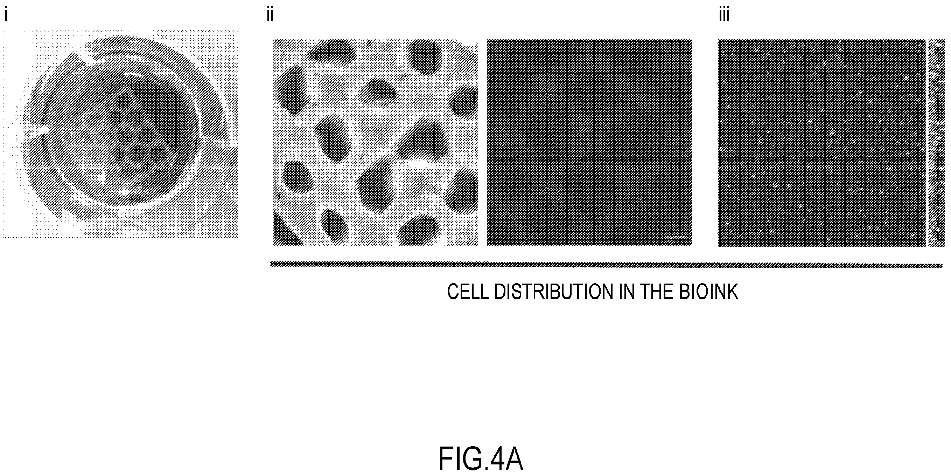

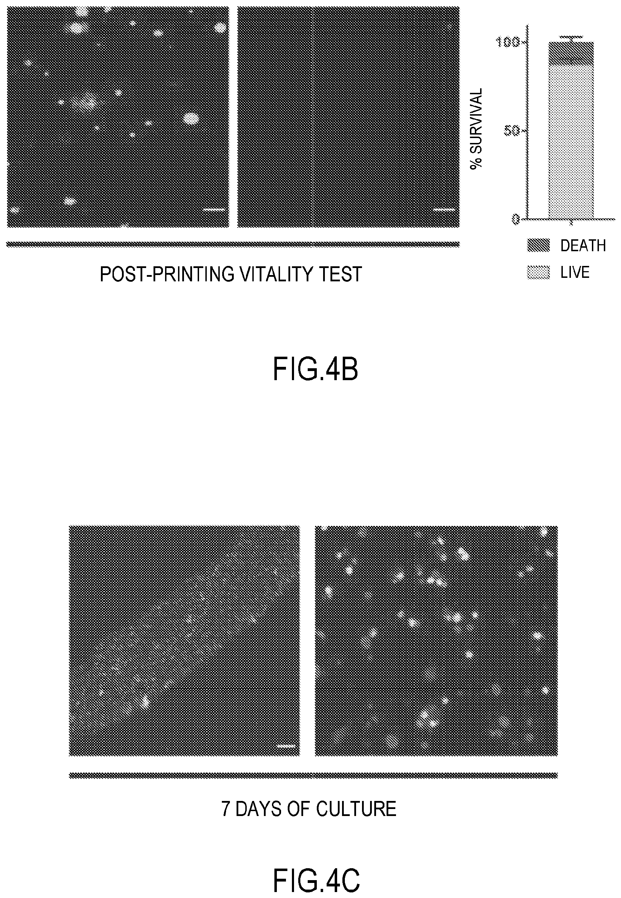

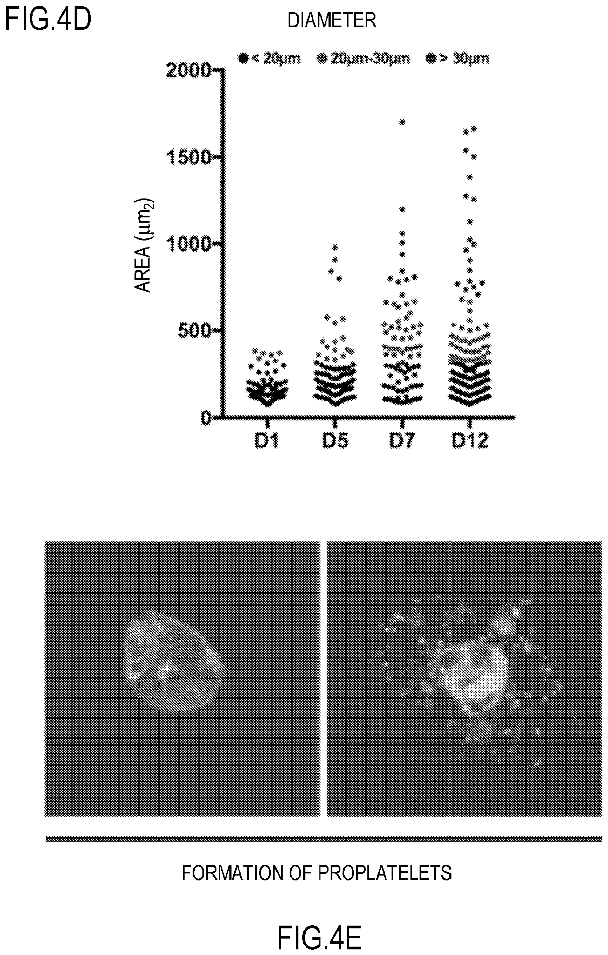

- Figure 4, panel A shows confocal microscopy images of the scaffold obtained by bioprinting using the bioink of the invention to which CD34⁺ hematopoietic stem cells (1x10⁶ cells/ml) are added. Panel B shows the z-stack confocal microscopy images after 24 hours post-printing showing a cell survival of about 90%. Panel C shows images showing the morphological change observed in cells that begin to enlarge and become polyploid during culture. Panel D shows a significant increase in the cell diameter, a typical feature of megakaryocyte differentiation and maturation, after 14 days of culture. Panel E shows the images following the marking of the actin cytoskeleton with TRITC-phalloidin which highlights the presence of a network of proplatelets within the ink.

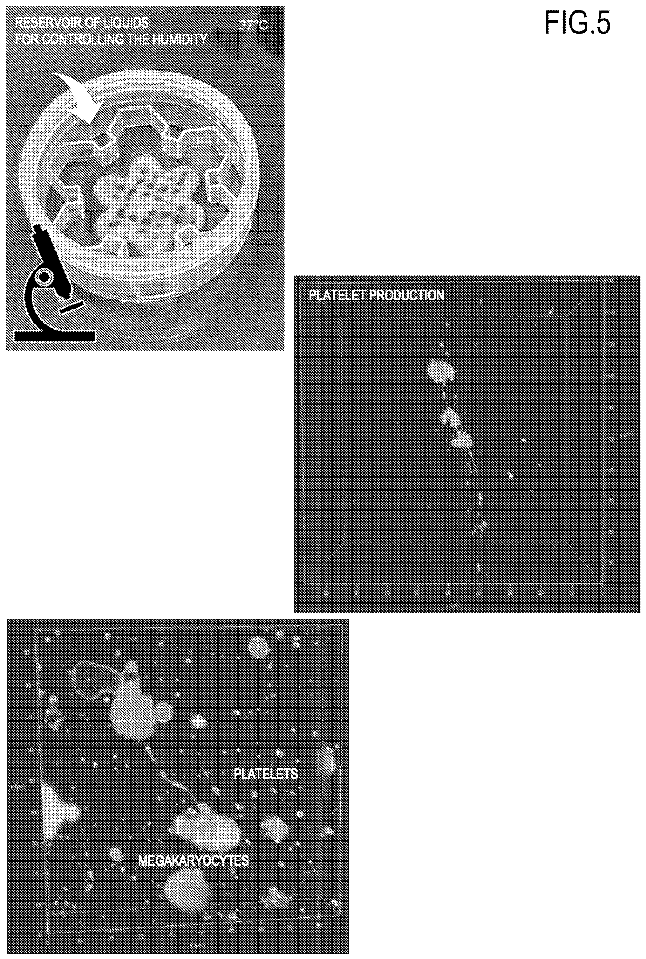

- Figure 5, shows the image of the customized culture chamber for microscopy viewing. The presence can be noted of a microscope glass at the bottom of the chamber. The space for the ink deposition and the subsequent culture of the scaffold and the wells for depositing the liquid reservoir for the maintenance of the humidified chamber, can be seen. 3D visualization of the culture obtained by real-time confocal microscopy shows a platelet-producing megakaryocyte. In addition, numerous platelets released into the ink are visible at the end of the culture. The megakaryocytes were pre-marked with an anti-CD61 antibody conjugated with fluorescein isothiocyanate before being included in the bioink and printed.

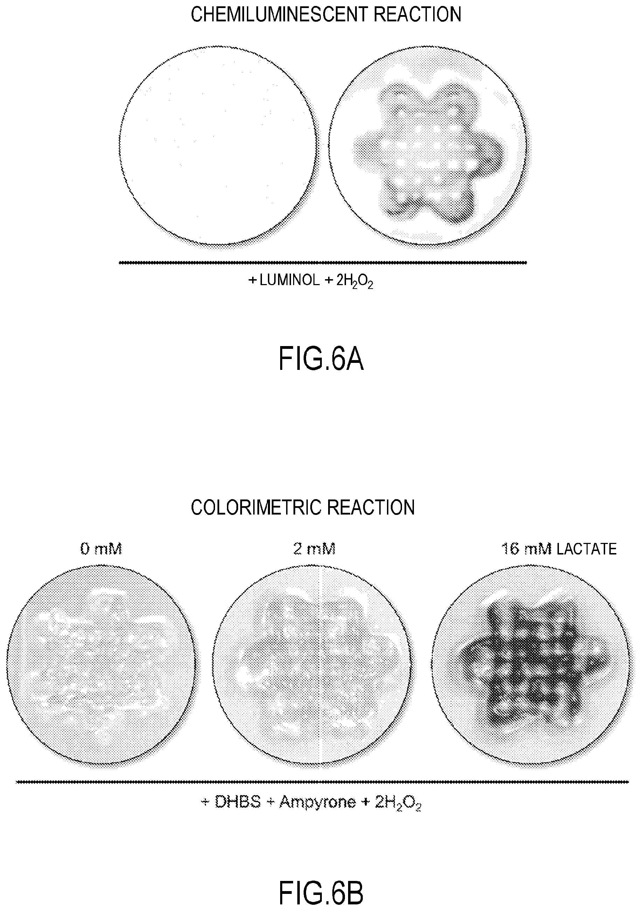

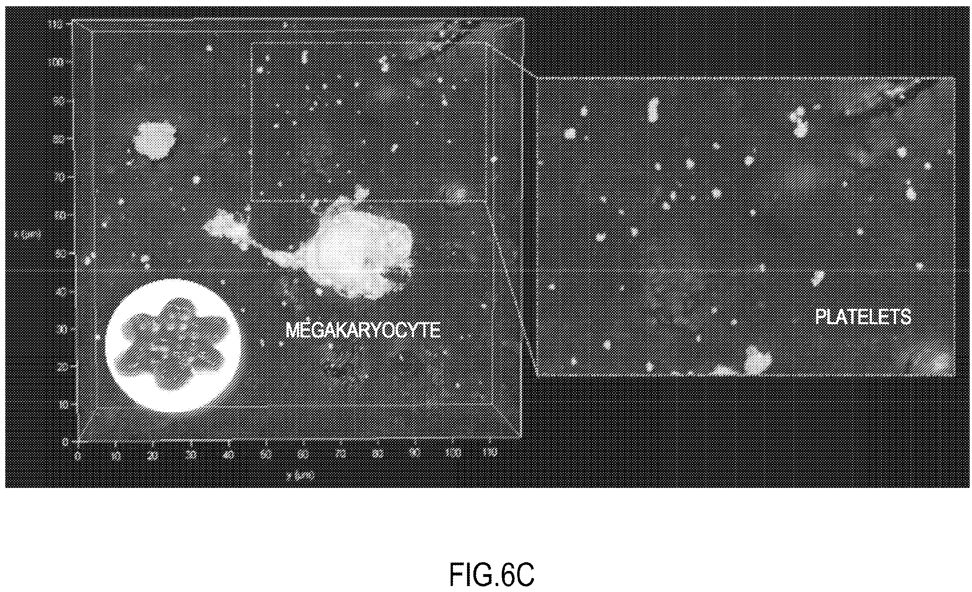

- Figure 6, panel A shows the result of the activation of a chemiluminescent reaction in a bioink functionalized with horseradish peroxidase (HRP). Panel B shows the colorimetric reaction developed in the HRP functionalized bioink and incubated with 2 or 16 mM lactate in the presence of lactate oxidase. Lactate-free incubation was used as a negative control. Panel C shows the colorimetric reaction activated by the lactate released by the same cells grown inside the 3D scaffold.

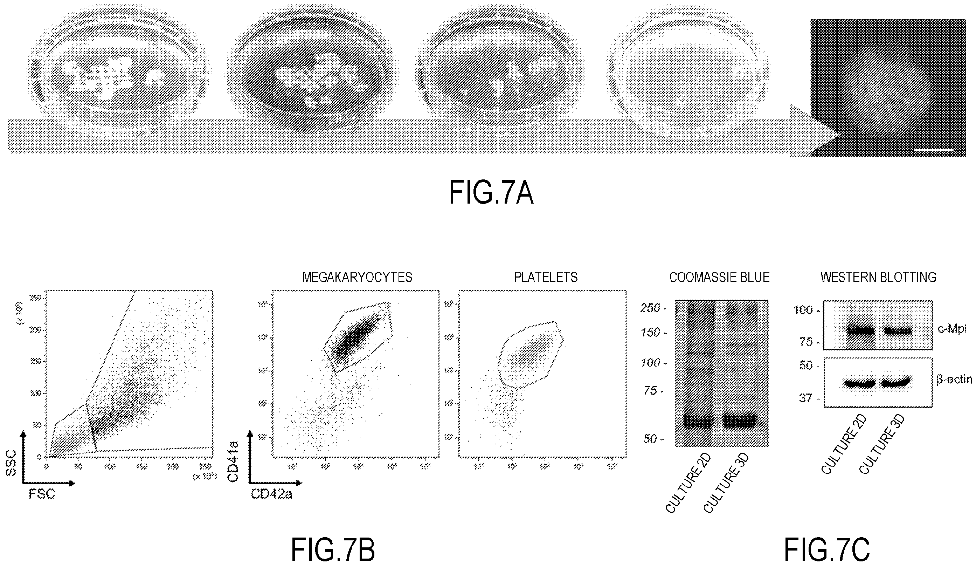

- Figure 7, panel A shows images of the dissolution process of the bioprinted scaffold with the formulation according to the invention. Dissolution was carried out after 2 weeks of culture. Released megakaryocytes were characterized by immunofluorescence microscopy after marking of the CD61 surface marker. Panel B shows the cyto-fluorometric flow analysis of the marking of membrane integrins CD41a and CD42a in cells recovered after dissolution of the scaffold. Panel C shows the result of an electrophoretic run of a protein lysate obtained from cells recovered after dissolution of the scaffold. The protein lysate obtained from cells grown in a classical culture plate was used for comparison. The Western blotting assay of the same protein lysates reveals the expression of the thrombopoietin receptor (TPO) comparable between cells grown under classic 2D culture conditions and cells obtained after ink dissolution.

The following non-limiting examples are now provided for a better illustration of the invention, in which different formulations of silk fibroin-based bioink were tested and compared, evaluating the quality and compatibility with the cells in the scaffolds obtained by 3D bioprinting.

### EXAMPLE 1: Preparation of fibroin-based bioink formulations

### Preparation of silk fibroin

The aqueous solution of silk fibroin was obtained from silkworm cocoons of the Bomby mori species deprived of the worms. The cocoons were boiled for 10, 20, 30, 40, 50 or 60 minutes in a solution of Na₂CO₃ in a weight/volume (w/v) ratio of 10g/4L. By varying the boiling time fibers having different molecular weights can be obtained. The fibers are rinsed for 20 minutes three times in ultra-pure water and dried overnight. The dry fibers were solubilized for 4 hours at 60°C in LiBr. The solubilized silk solution was subjected to dialysis with distilled water using a dialysis cassette with a threshold of 3.5 kDa for three days and changing the water for a minimum of eight times. The silk solution was finally centrifuged at a minimum rate of 2,000 rpm for 10-15 minutes to remove larger particles and stored at 4°C. The concentration of the silk solution was determined by drying a known volume of the solution overnight at 60°C and weighing the remaining solids. When necessary, the fibroin solution was concentrated in a dialysis cassette for 24 hours in a 20% PEG suspension.

### Silk fibroin-based bioink formulations

Various formulations were tested:
Formulation A: Silk fibroin ink A was prepared with a concentrated silk fibroin solution (35% w/v) modified with a saline buffer solution (2 mM NaCl, 6 mM KCl, 1 mM MgCl₂, 10 mM glucose, 10 mM HEPES) at pH 7.4 and gently sonicated by 3 pulses of 5 seconds at an amplitude of 20%. The ink is stored at 4°C for up to one month after preparation.

This composition made it possible to carry out the 3D printing of a singlelayer structure.

Formulation B (EMS according to the invention): The silk fibroin-based ink B was prepared with an 8% w/v silk fibroin solution. The solution was heated to 45°C. Type A gelatin (15% w/v) and sterile alginic acid (1% w/v) were incorporated in a 15:1 ratio, progressively under gentle magnetic stirring until complete dissolution, in order to respectively ensure the printability and cross-linking of the final form. A concentrated saline buffer solution is then slowly added to reach a final concentration of 2 mM NaCl, 6 mM KCl, 1 mM MgCl₂, Glucose was added to a final concentration of 10 mM. HEPES was added to a final concentration of 10 mM. Percoll^{®} was added to a final concentration of 2.5%. The pH was adjusted to 7.4. The solution was centrifuged for 3 minutes at 300xg at room temperature and stored at 4°C for up to two months after preparation. The ink was heated to 37°C before the cells were incorporated in the composition.

The printing efficiency was verified by comparing the composition with respect to the SG formulation (8% w/v silk fibroin, 15% w/v type A gelatin in saline solution) or SA (8% w/v silk fibroin, sterile alginic acid (1% w/v) in saline solution). Only with the SGA composition was it possible to 3D print a multi-layer structure (up to 20 layers) with stable junctions between the layers which, after crosslinking, remain intact even after repeated stretching and rewinding.

Fomulation C: This formulation is specific for 3D printing in a gel bath. The silk fibroin-based ink B was prepared with an 8% w/v silk fibroin solution. The solution was heated to 45°C. Sterile alginic acid (8% w/v) was gradually incorporated under gentle magnetic stirring until complete dissolution, in order to respectively ensure printability and crosslinking of the final form. A concentrated saline buffer solution is then slowly added to reach a final concentration of 2 mM NaCl, 6 mM KCl, 1 mM MgCl₂. Glucose was added to a final concentration of 10 mM. HEPES was added to a final concentration of 10 mM. The pH was adjusted to 7.4. The preparation was gently sonicated by 3 5-second pulses at an amplitude of 20%.

The solution was centrifuged for 3 minutes at 300xg at room temperature and stored at 4°C for up to one month after preparation. The ink was heated to 37° C before being added to the cells.

This composition made it possible to 3D print a multi-layer structure (up to 10 layers) having stable junctions between the layers that remain intact even after repeated stretching and rewinding.

All of the above formulations were tested in combination with different biomolecules, such as for example:
- components of the extracellular matrix (fibronectin, collagen, laminin);
- growth factors, preferably hematopoietic (TGF-β1, VEGF);
- cytokines and chemokines (TPO, GM-CSF, EPO, SDF-1α);
- drugs (TPO mimetics, ROCK inhibitors, kinase inhibitors);
- conjugated or unconjugated antibodies;
- enzymes and/or substrates and intermediates of enzymatic reactions;
- toxins.

All of the silk fibroin-based inks were sterilized by UV radiation prior to testing the cell cultures.

### EXAMPLE 2: Use of ink formulations in a bioprinting process

### Bioprinting process

3D models were produced using CAD software, processed with Slic3r or Cura to obtain the gCode which is then read by the bioprinter.

Different printing protocols were tested:
A) Formulation A was loaded into a 3ml cartridge fitted with a 27G nozzle and kept at 30°C. Printing was carried out directly in cell culture plates keeping the printer base at 37°C. The extrusion pressure was set at 5 kPa and the printing rate at 5 mm/s. The resulting scaffold was dried and sterilized by UV light before carrying out the cell culture tests. The structure is stable for at least 6 months at room temperature and for at least two weeks of culture at 37°C and 5% CO₂.
B) Formulation B was loaded into a 3ml cartridge fitted with an 18G, 20G or 22G nozzle and kept at 37°C in the printer. The printer base was kept at 16-20°C to allow the gelatin to solidify ensuring the stability of the 3D structure during the extrusion process. The extrusion pressure was set from 8 to 20 kPa and the printing rate from 6 to 12 mm/s. The scaffold was then immersed for a minimum of 10 minutes in a crosslinking solution containing 0.1 M CaCl₂ dissolved in a buffer solution containing 2 mM NaCl, 6 mM KCl, 1 mM MgCl₂, 5 mM glucose, 10 mM HEPES, under conditions sterile at room temperature, and finally immersed in a cell culture medium. During culture at 37°C and 5% CO₂, a fraction of the gelatin is immediately released into the medium, but the structure is kept stable for at least one month of culture.
C) Formulation C was bioprinted in a gel bath that supports ink deposition during the printing process. The bath consisted of an agarose mixture enriched with 0.1 M CaCl₂ to ensure immediate crosslinking. The bioink was loaded into a 3 ml cartridge equipped with a 25 G nozzle and printed at an extrusion pressure of 20 kPa and at a printing rate of 8 mm/s. After 10 minutes of incubation at 37°C, the plate was immersed in an 0.1 M CaCl₂ solution. The printed form was finally recovered and immersed in a cell culture medium. The structure was kept stable for up to two weeks of culture.

In all of the formulations tested, the removal of silk fibroin from the ink components negatively affects the duration of the final scaffold, demonstrating that fibroin plays a fundamental role in giving total stability to the structure (Figure 1).

Figure 1 shows the comparison between the printing performance of formulation B in the presence of silk, gelatin and alginate (SGA) compared with silk and alginate (SA) or silk and gelatin (SG). The analysis reveals the clearly superior profile in terms of printability, crosslinking and duration of the composition only in the SGA formulation, including silk fibroin, gelatin and alginate.

### Printability and mechanical properties of the bioink formulations tested

The printability of the ink was evaluated using a polyvalent bioprinter equipped with a thermoregulated head with a 3 ml piston cartridge. Stainless steel cylindrical or plastic conical nozzles were used

The tests were carried out by printing a square layer of 30x30x1 mm with a progressive rate from 360 mm/min to 720 mm/min and using an extrusion pressure of 8 kPa, 12 kPa, 16 kPa or 20 kPa. The printing lines were observed by bright-field microscopy using an Olympus IX53 (Olympus Deutschland GmbH, Hamburg, Germany) with a 4x objective for measuring the diameter. Measurements of the rheological properties were conducted using a HR-2 TA rotational rheometer with a plate diameter of 20 mm. The compression tests were conducted with an HR-2 TA rheometer. Hydrogels of formulation B were produced by distributing the fibroin inks in a 3D mould and then crosslinking for 10 minutes with an 0.1 M CaCl₂ solution. The disc produced had a diameter of ≈8 mm and a height of ≈3 mm. The samples were loaded between the parallel stainless steel plates. The upper plate was then lowered until a compression force sufficient for ensuring contact was exerted.

### Characteristics of the bioprinted scaffold

The overall morphology of the printed structure was observed by confocal microscopy (FV10i, Olympus, Germany) using the autofluorescence of silk under UV light or by marking with fluorescent dyes. The degradation rate of the bioprinted construct was determined by quantifying the weight reduction under culture conditions (37°C, 5% CO₂). In short, the freshly printed samples were weighed at the beginning and incubated in DMEM at 37°C for 7 days. At predetermined times, the samples of the bioprinted construct were dried and weighed.

### Cell sources

In order to test the efficiency and biocompatibility of silk fibroin inks, experiments were conducted with different cell sources, including human leukemia cell lines, hematopoietic stem cells, mature blood cell progenitors (i.e., megakaryocytes) and induced pluripotent stem cells:
1. Human leukemic cell lines: the samples were maintained in Dulbecco's modified Eagle medium, supplemented with 10% fetal bovine serum, 1% penicillin/streptomycin, 1% L-glutamine and 10% ng/mL GM-CSF. The cells were marked with CFSE fluorescent dye and combined with silk fibroin-based bioinks (formulation B) at a concentration of 0.5-4x10⁶ cells/ml. After printing multiple layers, the scaffolds were crosslinked and cultured for 2 weeks, with a change of medium/cytokines every 2 hours. The starting concentration was set at 1x10⁶ cells/ml.
   The cells however retained the capacity of proliferating which was assessed by counting the increasing number of cells/µm² by 3D reconstruction of the printed scaffold in the culture period.
2. Hematopoietic stem cells: human umbilical cord blood or peripheral blood hematopoietic progenitors were collected after issuing informed consent, according to the ethical requirements and principles of the Declaration of Helsinki. CD34⁺ or CD45⁺ hematopoietic stem cells were isolated by selection with immunomagnetic beads. The hematopoietic stem cells were marked with the CFSE dye and mixed with fibroin-based inks (formulation B) at a concentration of 1x10⁶ cells/ml. After printing multiple layers, the scaffolds were crosslinked and cultured for 2 weeks, with a change of medium/cytokines every 3 days. Alternatively, the cells were genetically engineered to express the GFP marker.
3. Mature blood cell progenitors: hematopoietic stem cells were cultured in a classical liquid medium containing cell-line specific cytokines. Once differentiated, the cells were mixed with the fibroin-based inks (formulation B) at a concentration of 1x10⁶ cells/ml. After printing multiple layers, the scaffolds were crosslinked and cultured for 1-2 weeks, with a change of medium/cytokins every 3 days.
   In the presence of thrombopoietin, the cells showed the formation of an invasive network of proplatelets, precursors of platelets, inside the ink, thus demonstrating that the 3D microenvironment provided is compatible with the functionality of blood cells.
4. Induced pluripotent stem cells: the samples derive from hematopoietic stem cell progenitors and differentiation is induced *in vitro* in the presence of a cocktail of cytokines.

Under all the conditions tested, the cell vitality after bioprinting was evaluated by the Live/Dead assay. The maximum intensity projection of five single random Z-stack fields, for each specimen under the microscope, was chosen for the calculations. The results demonstrate a cell survival of the printing process of 90% or more.

### RESULTS

### Mechanical properties and printability of silk fibroin-based ink

The silk fibroin ink (formulation B) was printed at 37°C and tested at different rates and using different nozzles (Figure 2, panels A-B). For cell printing, the variable parameters were the size of the nozzle, the extrusion pressure and the deposition rate of the ink (Figure 2, panel C). The amplitude of the variation of ink deposition is directly related to the variation of the rate and pressure, and shows a better resolution at 8-16 kPa in the printing-rate range of 480-720 mm/min.

Different configurations can therefore be produced depending on the desired final print resolution (Figure 2, panel C).

By comparing the ink in the presence of silk fibroin/gelatin/alginate (SGA) or gelatin/alginate (GA), it was observed that the 3D form was maintained only with SGA. The weight of both scaffolds fluctuated after 7 days and the phenomenon is related to the dispersion of the gelatin fraction at 37°C in the medium. The silk fibroin scaffold deposition however held a better form and was easier to manipulate (Figure 2, panels D-E).

In order to study the viscosity variations of the silk fibroin-based bioink that occur with printing, analyses were carried out using a rheometer. The analysis of the dependence of the modules on the temperature of the silk fibroin ink shows that the solution has viscoelastic properties with a critical gelling point in the temperature range of 28-30°C. The dependence of viscosity on the shear stress of the silk fibroin ink was measured at different temperatures and a stable behaviour was found below 30°C. The rigidity analysis was carried out by measuring the frequency sweeps of the silk fibroin ink after ionic crosslinking at 37°C. The storage modules were less than 1 kPa. The viscosity of the silk fibroin-based ink was studied in relation to the sudden switch of the shear stress, demonstrating a thixotropic behaviour. Furthermore, it takes at least 10 minutes to return to the initial viscosity and the process is completely reversible (Figure 2, panel F).

### Characteristics of 3D post-print constructs

SGA-type silk fibroin ink (formulation B) was loaded into the printer at 37°C under sterile conditions and a base square scaffold of 20x20x3mm with 15-20% internal reticular filling was printed using a 22 G nozzle, an extrusion pressure of 10 kPa and a printing rate of 10 mm/sec. The print bed was set at 16°C to allow a fast solidification of the ink after deposition on the plate. The scaffold was finally crosslinked with calcium ions for 15 minutes and subsequently immersed in culture medium to be kept under cell culture conditions (37°C, 5% CO₂). Upon optical microscopy, the printed forms were transparent and showed some fibrillar structures (Figure 2, panel G). The observation of a scaffold marked with Hoechst fluorescent dye in confocal microscopy showed the typical fibrillar organization of the polymerized silk fibroin (Figure 2, panel G).

As the diffusion coefficients of nutrients in the materials of 3D printed scaffolds are generally extremely low, the composition of the ink can have an important impact on their distribution within the printed scaffolds. The bioinks according to the invention (SGA - formulation B) allow the addition of a wide range of additives, including, but not limited to, cytokines, ECM (e.g. fibronectin), small molecules (e.g. ADP, ATP), fluorescent probes of intracellular compartments (e.g. cytoskeleton, organelles), biocompatible dyes, antibodies and enzymes to provide 3D printed scaffolds with additional functions. The dispersion of biocompatible dyes in the culture medium, for example, demonstrated a high retention of molecules within the silk fibroin ink (Figure 3, panel C).

The enzymatic activity of horseradish peroxidase integrated in a printed scaffold significantly allowed the emission of chemiluminescence in the presence of enzymatic substrates. Another advantage of the ink formulations according to the invention is that their use avoids the use of vulcanization methods of the printed scaffold that could reduce the biological activity of the additives integrated therein and/or of the cells themselves.

### Cell vitality and differentiation within 3D constructs

Bioprinting is a process that can lead cells to a condition of stress or even death. The pneumatic pressure applied in the cartridge and the rheological changes that occur inside the nozzle are parameters (among others) that have a significant impact on cell vitality.

The CD34⁺ hematopoietic stem cells were combined with silk fibroin ink at a concentration of 1x10⁶ cells/ml and bioprinted following the settings described above (Figure 4, panel A, i, ii).

Observation using confocal microscopy showed that the cells were well distributed within the scaffold and in a three-dimensional space (Figure 4, panel A, iii). 24 hours after post-printing, the scaffolds were marked with cell vitality/death probes and observed with z-stack confocal microscopy showing approximately 90% survival (Figure 4, panel B). The cells were kept in culture for 14 days during which the medium/cytokine was changed every 3 days. The scaffolds were observed by live microscopy at different times or fixed in a solution of 5% formaldehyde and 0.1 M CaCl₂ to effect marking of the cytoskeleton. After 7 days of culture, the cells are still homogeneously distributed in the scaffold and begin to enlarge and become polyploid (Figure 4. panel C). A significant increase in the cell diameter, a typical feature of megakaryocyte differentiation and maturation, was observed after 14 days of culture (Figure 4, panel D). Furthermore, the marking of the actin cytoskeleton with TRITC-phalloidin revealed the presence of a network of platelets within the printed structure, thus demonstrating that the 3D microenvironment provided is compatible with the function of megakaryocytes (Figure 4, panel E). Another noteworthy element is that the removal of silk fibroin from the components of the ink tested prevents long-term cell vitality and the culture of megakaryocytes, which are not capable of being differentiated to form proplatelets. All of these results show that the bioink formulation and bioprinting conditions are favourable for studying hematopoiesis and the formation of platelet.

### In situ analysis of the bioprinted cells:

The advantage of using fibroin for bioprinting is the transparency that allows easy optical accessibility for viewing in confocal microscopy and for detecting the signal of enzymatic reactions activated in the bioink itself.

The possibility of visualizing the culture in real-time was tested by bioprinting CD61⁺ hematopoietic progenitors pre-marked with antibody directed against the marker, conjugated with fluorochrome. The cells were combined with silk fibroin ink at a concentration of 1x10⁶ cells/ml and bioprinted following the settings described above. For visualization in confocal microscopy, the scaffold was printed inside a sterile culture chamber with a glass bottom for microscopy (Figure 5). 7 days after post-printing, the chamber was placed on the microscope stand and held at 37°C for a further 48 hours. Confocal reconstruction of the 3D scaffold shows the phases of the platelet production, from the elongation of the pseudopods to the release of platelets within the bioink, demonstrating the capacity of the scaffold of promoting and supporting the differentiation and function of hematopoietic cells (Figure 5). An advantage of printing pre-marked cells is the possibility of proceeding with the direct visualization of the sample without the need for adding additional reagents.

A further bioactivity test was performed by incorporating horseradish peroxidase (HRP) into the ink to verify the stability and functionality of the enzyme in the printed tissue. The formulation was printed at 37°C and kept in culture for 2 weeks. Incubation with luminol and hydrogen peroxide triggered a chemiluminescence reaction. The measurement of the variation in intensity of the light emission was detected only by the HRP-functionalized bioink, with respect to the control bioink (HRP-free formulation B) (Figure 6A).

The HRP activity was also tested through a colorimetric reaction. The B+HRP formulation was added to lactate and incubated in a solution containing lactate oxidase, DHBS and 4-AAP. The resulting reaction produces pyruvate and hydrogen peroxide. The latter reacts with HRP causing a change in the colour of the printed scaffold, proportional to the quantity of lactate present in the scaffold (Figure 6B). In order to validate the capacity of the B+HRP formulation of revealing the presence of molecules released by the cells cultured in the scaffold, CD61⁺ cells were added to the ink at a concentration of 1x10⁶ cells/ml and bioprinted following the settings described above. After 7 days of culture the scaffold was immersed in a solution containing lactate oxidase, DHBS and 4-AAP. The staining reaction takes place within a few minutes (Figure 6). When the cells are printed in the presence of lactate dehydrogenase inhibitor, the amount of lactate detectable decreases significantly reducing the colorimetric signal. These results extend the usefulness of adopting bioink not only as a biological stabilizer of the enzymatic activity of the molecules added to the formulation for carrying out chemical and enzymatic assays but also for detecting multi-analytes produced by the hematopoietic cells.

### Dissolution of the scaffold and recovery of bioprinted cells:

3D cultured hematopoietic cells can be used for biochemical and molecular studies. Furthermore, there is increasing interest in the possibility of using the cells in functional assays or for clinical use. For this purpose, the cells must be able to be recovered from the bioprinted scaffold for subsequent uses.

CD61⁺ cells were combined with silk fibroin ink at a concentration of 1x10⁶ cells/ml and bio-printed following the settings described above. After 7 days of culture the scaffold was immersed in a dissolution solution (according to the invention) (Figure 7A). The cells were recovered in liquid solution and analyzed by immunofluorescence microscopy (Figure 7A) and flow cytometry (Figure 7B). The cell lysate of the same cells was subjected to an electrophoretic run. The staining of the bands using Coomassie blue reveals a protein abundance comparable to that of a sample of cells obtained from a classic 2D culture, demonstrating efficiency in the recovery of the sample. The protein sample can be used for subsequent biochemical analyses (e.g. Western blotting) (Figure 7C).

### EXAMPLE 3:

### Use of the ink formulations for real-time visualization of in vitro platelet production

In order to test the possibility of viewing the bioprinted cells in real-time without fixing or manipulating the 3D printed scaffold, customized culture chambers having the same shape as the scaffold were created. Both of the 3D models of the scaffold and the culture chamber were created using CAD software. The perfusion chamber was printed using a biocompatible resin by means of the stereolithography technique. The bottom of the chamber was sealed using a confocal microscopy glass (0.17 mm) (Figure 5). Formulation B of the bioink was printed inside the chamber. Before being injected into the bioink, the hematopoietic progenitors were marked with anti-CD61 antibody conjugated with a fluorescent marker. After bioprinting the cells in formulation B, the scaffold was crosslinked and subsequently incubated in a culture medium. Visualization of the cells during the production of the platelets can take place at different times according to the experimental requirements, by removing the culture medium and positioning the chamber directly on the stand of the confocal microscope, maintained at 37°C. During visualization, the maintenance of the pH of the scaffold is ensured by the presence of the buffer system (HEPES). The humidified environment is guaranteed by the presence of a liquid reservoir in the chamber itself.

### EXAMPLE 4: Use of the ink formulations for the enzymatic dosage of lactate released by the cells during culture

In order to test the bioactivity of the bioink, the possibility of functionalizing formulation B with the horseradish peroxidase (HRP) enzyme was tested. For this purpose, 110 U/mL of HRP were added to formulation B. The B+HRP formulation was printed and, after crosslinking, incubated for 14 days in a culture medium.

The bioactivity was evaluated after several days of culture by activating a reaction leading to chemiluminescence in the presence of luminol and hydrogen peroxide. The chemiluminescent signal was acquired using a chemiluminescence detector. In all the conditions tested, the enzymatic activity was detectable.

The B+HRP formulation was also tested in the presence of hematopoietic progenitors for dosing molecules released during the 3D culture by the cells themselves, without the need for manipulating the scaffold or resoring to the preparation of enzymatic assays of the culture mediums. In particular, the release of lactate, a product of glycolysis, indicative of an active cellular metabolism and whose variation can be indicative of alterations in the platelet production, was analyzed. For this purpose, the reagents: 20 U/mL of lactate oxidase; 600 ng/mL of sodium 3,5-dichloro-2-hydroxybenzenesulfonate (DHBS), 100 ng/mL of 4-aminoantipyrine (4-AAP) were added to the B+HRP formulation.. Alternatively, the reagents can be added directly to the culture medium and diffused in the bioink at the time of the assay. In both cases, the bioactivity of the preparation was confirmed by the activation of a colorimetric reaction following incubation with lactate (2 or 16 mM). Lactate-free incubation was used as a negative control.

The presence of increasing the concentrations of lactate released by the cells was detected during the culture of the bioprinted 3D scaffold. The specificity of the reaction was confirmed by treating the sample with the lactate dehydrogenase inhibitor.

### EXAMPLE 5:

### Dissolution of the bioprinted scaffold and use of the cells recovered from 3D culture

In order to test the possibility of recovering the cells from the bioink after 3D culture, and using them for functional, biochemical or molecular studies, hematopoietic progenitors were bioprinted with formulation B.

After 7 days of culture, the scaffold was incubated in a dissolution solution (according to the invention), at 37°C for 20 minutes.

The cells were then centrifuged at 1,200 rpm for 10 minutes, washed in a physiological solution and used in the experimental assays.

The morphological parameters and the expression of surface antigens of the megakaryocyte lineage were evaluated by immunofluorescence microscopy and flow cytometry after incubation with specific antibodies. The protein lysate was dosed by spectrophotometry, tested by an electrophoretic run in 8% polyacrylamide gel and visualized by marking with Coomassie Blue or Western blotting.

### BIBLIOGRAPHY

1. Murphy SV, Atala A. 3D bioprinting of tissues and organs. Nat Biotechnol. 2014 Aug;32(8):773-85. eng. Epub 2014/08/06. doi:10.1038/nbt.2958. Cited in: Pubmed; PMID 25093879.
2. Lim KS, Levato R, Costa PF, Castilho MD, Alcala-Orozco CR, van Dorenmalen KMA, Melchels FPW, Gawlitta D, Hooper GJ, Malda J, Woodfield TBF. Bio-resin for high resolution lithography-based biofabrication of complex cell-laden constructs. Biofabrication. 2018 May 11;10(3):034101. eng. Epub 2018/04/26. doi:10.1088/1758-5090/aac00c. Cited in: Pubmed; PMID 29693552.
3. Mandrycky C, Wang Z, Kim K, Kim DH. 3D bioprinting for engineering complex tissues. Biotechnol Adv. 2016 Jul-Aug;34(4):422-434. eng. Epub 2016/01/03. doi:10.1016/j.biotechadv.2015.12.011. Cited in: Pubmed; PMID 26724184.
4. Wang Z, Kumar H, Tian Z, Jin X, Holzman JF, Menard F, Kim K. Visible Light Photoinitiation of Cell-Adhesive Gelatin Methacryloyl Hydrogels for Stereolithography 3D Bioprinting. ACS Appl Mater Interfaces. 2018 Aug 15;10(32):26859-26869. eng. Epub 2018/07/20. doi:10.1021/acsami.8b06607. Cited in: Pubmed; PMID 30024722.
5. Ashammakhi N, Ahadian S, Xu C, Montazerian H, Ko H, Nasiri R, Barros N, Khademhosseini A. Bioinks and bioprinting technologies to make heterogeneous and biomimetic tissue constructs. Mater Today Bio. 2019 Jan;1:100008. eng. Epub 2020/03/12. doi:10.1016/j.mtbio.2019.100008. Cited in: Pubmed; PMID 32159140.
6. Morgan FLC, Moroni L, Baker MB. Dynamic Bioinks to Advance Bioprinting. Adv Healthc Mater. 2020 Aug;9(15):e1901798. eng. Epub 2020/02/27. doi:10.1002/adhm.201901798. Cited in: Pubmed; PMID 32100963.
7. Chawla S, Midha S, Sharma A, Ghosh S. Silk-Based Bioinks for 3D Bioprinting. Adv Healthc Mater. 2018 Apr;7(8):e1701204. eng. Epub 2018/01/24. doi:10.1002/adhm.201701204. Cited in: Pubmed; PMID 29359861.
8. Morrison SJ, Scadden DT. The bone marrow niche for haematopoietic stem cells. Nature. 2014 Jan;505(7483):327-34. eng. doi:10.1038/nature12984. Cited in: Pubmed; PMID 24429631.
9. Ribeiro-Filho AC, Levy D, Ruiz JLM, Mantovani MDC, Bydlowski SP. Traditional and Advanced Cell Cultures in Hematopoietic Stem Cell Studies. Cells. 2019 Dec 12;8(12). eng. Epub 2019/12/18. doi:10.3390/cells8121628. Cited in: Pubmed; PMID 31842488.
10. Wilkinson AC, Igarashi KJ, Nakauchi H. Haematopoietic stem cell self-renewal in vivo and ex vivo. Nat Rev Genet. 2020 Sep;21(9):541-554. eng. Epub 2020/05/30. doi:10.1038/s41576-020-0241-0. Cited in: Pubmed; PMID 32467607.
11. Méndez-Ferrer S, Michurina TV, Ferraro F, Mazloom AR, Macarthur BD, Lira SA, Scadden DT, Ma'ayan A, Enikolopov GN, Frenette PS. Mesenchymal and haematopoietic stem cells form a unique bone marrow niche. Nature. 2010 Aug;466(7308):829-34. eng. doi:10.1038/nature09262. Cited in: Pubmed; PMID 20703299.
12. Pallotta I, Lovett M, Rice W, Kaplan DL, Balduini A. Bone marrow osteoblastic niche: a new model to study physiological regulation of megakaryopoiesis. PLoS One. 2009;4(12):e8359. eng. doi:10.1371/journal.pone.0008359. Cited in: Pubmed; PMID 20027303.
13. Greenbaum A, Hsu YM, Day RB, Schuettpelz LG, Christopher MJ, Borgerding JN, Nagasawa T, Link DC. CXCL12 in early mesenchymal progenitors is required for haematopoietic stem-cell maintenance. Nature. 2013 Mar;495(7440):227-30. eng. doi:10.1038/nature11926. Cited in: Pubmed; PMID 23434756.
14. Dexter TM, Allen TD, Lajtha LG. Conditions controlling the proliferation of haemopoietic stem cells in vitro. J Cell Physiol. 1977 Jun;91(3):335-44. eng. Epub 1977/06/01. doi:10.1002/jcp.1040910303. Cited in: Pubmed; PMID 301143.
15. Zhang CC, Lodish HF. Cytokines regulating hematopoietic stem cell function. Curr Opin Hematol. 2008 Jul;15(4):307-11. eng. Epub 2008/06/10. doi:10.1097/MOH.0b013e3283007db5. Cited in: Pubmed; PMID 18536567.

## Claims

1. A bioink formulation comprising:
| | |
|---|---|
| - silk fibroin | 0.5%-35% w/v |
| - gelatin or derivatives thereof | 5-20% w/v |
| - alginic acid or derivatives thereof | 0.5-2% w/v |
| - glucose | 3-4% w/v |
| - controlled density solution | 1-3% w/v |
| - buffer | 4-6% w/v |
| - saline buffer solution | up to 100% |
wherein the gelatin and alginic acid or derivatives thereof are present in a reciprocal ratio ranging from about 10:1 to about 20:1;
wherein the silk fibroin and gelatin are present in a reciprocal ratio ranging from about 1:5 to about 1:0.5;
suitable for extrusion with hematopoietic lineage cells.

2. The bioink formulation according to claim 1, further comprising a cell component selected from the group consisting of progenitor cells of the myeloid or lymphoid lineage, stem cells or differentiated cells of the hematopoietic lineage selected from the group consisting of monocytes, macrophages, granulocytes, neutrophils, basophils, eosinophils, red cells, megacaryocytes, platelets, dendritic cells, T lymphocytes, B lymphocytes, NK cells, fibroblasts, mesenchymal stem cells, endothelial cells.

3. The bioink formulation according to any of claims 1-2, wherein said cells are present at a concentration within the range of 0.5-4x10⁶ cells/ml, preferably 1x10⁶ cells/ml.

4. The bioink formulation according to any of claims 1-3, wherein the gelatin is type A or type B.

5. The bioink formulation according to any of claims 1-4, wherein the derivatives of alginic acid are salts with alkaline and alkaline earth metals selected from the group consisting of sodium alginate, calcium alginate, magnesium alginate, potassium alginate and alginate methacrylate.

6. The bioink formulation according to any of claims 1-5, wherein the controlled density solution is a defined density solution selected from the group consisting of , , colloidal silica, copolymers, hydrophilic polysaccharides or synthetic high-molecular-weight polymers.

7. The bioink formulation according to any of claims 1-6, wherein the buffer is organic or mineral selected from the group consisting of: HEPES, MES, ADA, ACES, PIPES, MOPSO, Bis-6Tris Propane, BES, MOPS, TES, DIPSO, MOBS, TAPSO, HEPPSO, POPSO, EPPS (HEPPS), Tricine, Gly-Gly, Bicine, HEPBS, TAPS, AMPD, TABS, AMPSO, CHES, CAPSO, AMP, CAPS and CABS.

8. The bioink formulation according to any of claims 1-7, further comprising at least one fluorescent marker, a contrast agent, a luminescent agent, a radiopaque agent, a radioactive element or combinations thereof.

9. The bioink formulation according to any of claims 1-8, further **characterized in that** it is sterile.

10. The bioink formulation according to any of claims 1-9, further **characterized in that** it is at a temperature of 37°C.

11. Use of a bioink formulation according to any of claims 1-10, in a bioprinting process.

12. Use of a bioink formulation according to any of claims 2-10, for the production of scaffolds comprising progenitor cells of the myeloid or lymphoid lineage, stem cells or differentiated cells of the hematopoietic lineage.

13. A bioprinting method for the producton of a 3D scaffold, comprising the following steps:
a) bioprinting of the bioink formulation according to any of claims 2-10 through a nozzle having a caliber ranging from 18G to 27G at an extrusion pressure within the range of 5-200 kPa and a printing rate within the range of 5-1,000 mm/min on a substrate to obtain a mono- or multi-layer 3D scaffold;
b) soaking the 3D scaffold obtained according to step a) in a reticulating solution;
c) adding the cell culture medium to the scaffold and optionally growth factors to promote cell differentiation.

14. The bioprinting method for the producton of a 3D scaffold according to claim 13, wherein the extrusion pressure ranges from 5kPa to 20 kPa and the printing rate is within the range of 400-1,000 mm/min.

15. A scaffold obtainable by means of a bioprinting method of the bioink formulation according to any of claims 1-10.

16. A scaffold obtainable by means of a bioprinting method of the bioink formulation according to any of claims 13-14.

17. The scaffold according to any of claims 15-16, further comprising one or more molecules selected from the group consisting of:
- components of the extracellular matrix such as proteoglycans, hyaluronic acid, collagens, elastin, fibronectin, fibrin, fibrinogen, laminin, thrombospondin;
- growth factors, cytokines and chemokins, preferably hematopoietic, such as interleukins, CSF-1, G-CSF, GM-CSF, SCF, FLT3-L, TPO, EPO, SDF-1α; TGF-β1, TNFα, VEGF, FGF, Notch ligands, WNT, Angiopoietin-1, BMP, IGF-2 and fragments or variants thereof;
- antigens, conjugated or unconjugated antibodies, or their fragments such as CDRs or epitopes;
- hormones, such as insulin, glucagon, triiodothyronine, thyroxine, or their antagonists;
- drugs or prodrugs, such as TPO mimetics, TPO receptor agonists, tyrosine kinase receptor agonists, tyrosine kinase receptor inhibitors, Rho kinase inhibitors (ROCK), kinase inhibitors, aryl hydrocarbon receptor antagonists, chemotherapeutic agents;
- nucleic acids, such as DNA, RNA, siRNA, RNAi and microRNA, plasmids, lentiviruses, CRISPR;
- enzymes and/or intermediates of enzymatic reactions;
- toxins.

18. A method for dissolving the scaffold obtainable by means of a bioprinting method of the bioink formulation according to any of claims 1-10, wherein the method comprises preparing the scaffold by means of the method of claim 13 and further comprises using a dissolution solution of the scaffold comprising sodium citrate, collagenase, alginate lyase and a source of glucose.

19. The method according to claim 18, wherein the dissolution solution of the scaffold comprises:
- sodium citrate 15 mM
- collagenase 10 U / mL
- alginate lyase 5 U / mL
- 5 mM glucose
- saline buffer solution

20. Use of the scaffold according to any of claims 15-17, as an *ex vivo* model for supporting hematopoiesis or for the production of platelets and blood cells.

21. A scaffold according to any of claims 15-17, for use as a surgical implant for supporting *in vivo* hematopoiesis or the production of platelets and blood cells.

## Patentansprüche

1. Eine Biotintenformulierung, umfassend:
| | |
|---|---|
| - Seidenfibroin | 0,5%-35% Gewicht pro Volumen |
| - Gelatine oder deren Derivate | 5-20% Gewicht pro Volumen |
| - Alginsäure oder deren Derivate | 0,5-2% Gewicht pro Volumen |
| - Glukose | 3-4% Gewicht pro Volumen |
| - Lösung mit kontrollierter Dichte | 1-3% Gewicht pro Volumen |
| - Puffer | 4-6% Gewicht pro Volumen |
| - Salzpufferlösung | bis zu 100% |
wobei die Gelatine und die Alginsäure oder deren Derivate in einem gegenseitigen Verhältnis von etwa 10:1 bis etwa 20:1 vorhanden sind;
wobei das Seidenfibroin und die Gelatine in einem gegenseitigen Verhältnis von etwa 1:5 bis etwa 1:0,5 vorhanden sind;
geeignet für die Extrusion mit hämatopoetischen Stammzellen.

2. Biotintenformulierung nach Anspruch 1 ferner umfassend eine Zellkomponente, die aus der Gruppe bestehend aus Vorläuferzellen der myeloischen oder lymphoiden Abstammungslinie, Stammzellen oder differenzierten Zellen der hämatopoetischen Abstammungslinie, die aus der Gruppe bestehend aus Monozyten, Makrophagen, Granulozyten, Neutrophilen, Basophilen, Eosinophilen, Erythrozyten, Megakaryozyten, Thrombozyten, dendritischen Zellen, T-Lymphozyten, B-Lymphozyten, NK-Zellen, Fibroblasten, mesenchymalen Stammzellen und Endothelzellen ausgewählt werden, ausgewählt wird..

3. Biotintenformulierung nach einem der Ansprüche 1 bis 2, wobei die Zellen in einer Konzentration in einem Bereich von 0,5 bis 4x10⁶ Zellen/ml, vorzugsweise 1x10⁶ Zellen/ml, vorhanden sind.

4. Bioitinteformulierung nach einem der Ansprüche 1 bis 3, wobei die Gelatine vom Typ A oder Typ B ist.

5. Biotintenformulierung nach einem der Ansprüche 1 bis 4, wobei die Derivate der Alginsäure Salze mit Alkali- und Erdalkalimetallen sind, die aus der Gruppe bestehend aus Natriumalginat, Calciumalginat, Magnesiumalginat, Kaliumalginat und Alginatmethacrylat ausgewählt werden.

6. Biotintenformulierung nach einem der Ansprüche 1 bis 5, wobei die Lösung mit kontrollierter Dichte eine Lösung mit definierter Dichte ist, die aus der Gruppe bestehend aus kolloidalem Siliziumdioxid, Copolymeren, hydrophilen Polysacchariden oder synthetischen hochmolekularen Polymeren ausgewählt wird.

7. Biotintenformulierung nach einem der Ansprüche 1-6, wobei der Puffer organisch oder mineralisch ist, der aus der Gruppe bestehend aus: HEPES, MES, ADA, ACES, PIPES, MOPSO, Bis-6Tris Propan, BES, MOPS, TES, DIPSO, MOBS, TAPSO, HEPPSO, POPSO, EPPS (HEPPS), Tricine, Gly-Gly, Bicine, HEPBS, TAPS, AMPD, TABS, AMPSO, CHES, CAPSO, AMP, CAPS und CABS ausgewählt wird.

8. Die Biotintenformulierung nach einem der Ansprüche 1 bis 7 ferner umfassend mindestens einen Fluoreszenzmarker, ein Kontrastmittel, ein Lumineszenzmittel, ein Röntgenopakmittel, ein radioaktives Element oder Kombinationen davon.

9. Die Biotintenformulierung nach einem der Ansprüche 1 bis 8, **dadurch gekennzeichnet, dass** sie steril ist.

10. Biotintenformulierung nach einem der Ansprüche 1 bis 9, ferner **dadurch gekennzeichnet, dass** sie eine Temperatur von 37 °C aufweist.

11. Verwendung einer Biotintenformulierung nach einem der Ansprüche 1-10 in einem Bioprinting-Verfahren.

12. Verwendung einer Biotintenformulierung nach einem der Ansprüche 2-10 zur Herstellung von dreidimensionalen Gerüstbauen umfassend Vorläuferzellen der myeloischen oder lymphoiden Abstammungslinie, Stammzellen oder differenzierte Zellen der hämatopoetischen Abstammungslinie.

13. Bioprinting-Verfahren zur Herstellung einer 3D-Gerüstbau umfassend die folgenden Schritte:
a) Bioprinting der Biotintenformulierung nach einem der Ansprüche 2-10 durch eine Düse aufweisend einen Kaliber in einem Bereich von 18G bis 27G bei einem Extrusionsdruck in einem Bereich von 5-200 kPa und einer Druckgeschwindigkeit in einem Bereich von 5-1.000 mm/min auf ein Substrat, um eine einschichtige- oder mehrschichtige 3D-Gerüstbau zu erhalten;
b) Einweichen der 3D-Gerüstbau die nach Schritt a) erhalten wird in einer netzartigen Lösung;
c) Zugeben des Zellkulturmediums zu der Gerüstbau und optional Wachstumsfaktoren zum Fördern der Zelldifferenzierung.

14. Bioprinting-Verfahren zur Herstellung einer 3D- Gerüstbau nach Anspruch 13, wobei der Extrusionsdruck in einem Bereich von 5 kPa bis 20 kPa liegt und die Druckgeschwindigkeit in einem Bereich von 400-1.000 mm/min liegt.

15. Gerüstbau, die durch ein Bioprinting-Verfahren der Biotintenformulierung nach einem der Ansprüche 1-10 erhalten werden kann.

16. Gerüstbau, die durch ein Bioprinting-Verfahren der Biotintenformulierung nach einem der Ansprüche 13-14 erhalten werden kann.

17. Gerüstbau nach einem der Ansprüche 15-16, ferner umfassend ein oder mehrere Moleküle, die aus der Gruppe bestehend aus:
- Bestandteilen der extrazellulären Matrix wie Proteoglykane, Hyaluronsäure, Kollagene, Elastin, Fibronektin, Fibrin, Fibrinogen, Laminin und Thrombospondin;
- Wachstumsfaktoren, Zytokine und Chemokine, vorzugsweise hämatopoetische, wie Interleukine, CSF-1, G-CSF, GM-CSF, SCF, FLT3-L, TPO, EPO, SDF-1α; TGF-β1, TNFα, VEGF, FGF, Notch-Liganden, WNT, Angiopoietin-1, BMP, IGF-2 und Fragmente oder Varianten davon;
- Antigene, konjugierte oder unkonjugierte Antikörper oder deren Fragmente wie CDR oder Epitope;
- Hormone wie Insulin, Glucagon, Trijodthyronin, Thyroxin oder deren Antagonisten;
- Arzneimittel oder Prodrugs, wie TPO-Mimetika, TPO-Rezeptor-Agonisten, Tyrosinkinase-Rezeptor-Agonisten, Tyrosinkinase-Rezeptor-Inhibitoren, Rho-Kinase-Inhibitoren (ROCK), Kinase-Inhibitoren, Aryl-Kohlenwasserstoff-Rezeptor-Antagonisten, Chemotherapeutika;
- Nukleinsäuren, wie DNA, RNA, siRNA, RNAi und microRNA, Plasmide, Lentiviren, CRISPR;
- Enzyme und/oder Zwischenprodukte von enzymatischen Reaktionen;
- Toxine, ausgewählt werden.

18. Verfahren zum Auflösen der Gerüstbau, das mittels eines Bioprinting-Verfahrens der Biotintenformulierung nach einem der Ansprüche 1-10 erhalten werden kann, wobei das Verfahren das Herstellen der Gerüstbau mittels des Verfahrens nach Anspruch 13 umfasst und ferner die Verwendung einer Auflösungslösung der Zellenstützstruktur umfasst, die Natriumcitrat, Collagenase, Alginatlyase und eine Glukosequelle umfasst.

19. Verfahren nach Anspruch 18, wobei die Auflösungslösung der Gerüstbau umfasst:
- Natriumcitrat 15 mM
- Kollagenase 10 U / mL
- Alginatlyase 5 U / mL
- 5 mM Glukose
- Salzpufferlösung

20. Verwendung der Gerüstbau nach einem der Ansprüche 15-17 als Ex-vivo-Modell zum Fördern der Hämatopoese oder zur Produktion von Blutplättchen und Blutzellen.

21. Gerüstbau nach einem der Ansprüche 15-17 zur Verwendung als chirurgisches Implantat zum Fördern der In-vivo-Hämatopoese oder der Produktion von Blutplättchen und Blutzellen.

## Revendications

1. Formule de bio-encre comprenant:
| | |
|---|---|
| - de la fibroïne de soie | de 0,5 % à 35 % p/v |
| - de la gélatine ou ses dérivés | de 5 à 20 % p/v |
| - de l'acide alginique ou ses dérivés | de 0,5 à 2 % p/v |
| - du glucose | de 3 à 4 % p/v |
| - une solution à densité contrôlée | de 1 à 3 % p/v |
| - un tampon | de 4 à 6 % p/v |
| - une solution tampon saline | jusqu'à 100 % |
dans laquelle la gélatine et l'acide alginique ou leurs dérivés sont présents dans un rapport réciproque allant d'environ 10:1 à environ 20:1 ;
dans laquelle la fibroïne de soie et la gélatine sont présentes dans un rapport réciproque allant d'environ 1:5 à environ 1:0,5 ;
convient à l'extrusion de cellules de la lignée hématopoïétique.

2. Formulation de bio-encore selon la revendication 1, comprenant en outre un composant cellulaire choisi dans le groupe constitué de cellules progénitrices de la lignée myéloïde ou lymphoïde, de cellules souches ou de cellules différenciées de la lignée hématopoïétique choisies dans le groupe constitué de monocytes, macrophages, granulocytes, neutrophiles, basophiles, éosinophiles, globules rouges, mégacaryocytes, plaquettes, cellules dendritiques, lymphocytes T, lymphocytes B, cellules NK, fibroblastes, cellules souches mésenchymateuses, cellules endothéliales.

3. Formulation de bio-encre selon l'une quelconque des revendications 1 à 2, dans laquelle lesdites cellules sont présentes à une concentration comprise entre 0,5 et 4x10⁶ cellules/ml, de préférence 1x10⁶ cellules/ml.

4. Formulation de bio-encre selon l'une quelconque des revendications 1 à 3, dans laquelle la gélatine est de type A ou de type B.

5. Formulation de bio-encre selon l'une quelconque des revendications 1 à 4, dans laquelle les dérivés de l'acide alginique sont des sels de métaux alcalins et alcalino-terreux choisis dans le groupe constitué par l'alginate de sodium, l'alginate de calcium, l'alginate de magnésium, l'alginate de potassium et le méthacrylate d'alginate.

6. Formulation de bio-encre selon l'une quelconque des revendications 1 à 5, dans laquelle la solution à densité contrôlée est une solution à densité définie choisie dans le groupe constitué par la silice colloïdale, les copolymères, les polysaccharides hydrophiles ou les polymères synthétiques à poids moléculaire élevé.

7. Formulation de bio-encre selon l'une quelconque des revendications 1 à 6, dans laquelle le tampon est organique ou minéral choisi dans le groupe constitué de : HEPES, MES, ADA, ACES, PIPES, MOPSO, Bis-6Tris Propane, BES, MOPS, TES, DIPSO, MOBS, TAPSO, HEPPSO, POPSO, EPPS (HEPPS), Tricine, Gly-Gly, Bicine, HEPBS, TAPS, AMPD, TABS, AMPSO, CHES, CAPSO, AMP, CAPS et CABS.

8. Formulation de bio-encre selon l'une quelconque des revendications 1 à 7, comprenant en outre au moins un marqueur fluorescent, un agent de contraste, un agent luminescent, un agent radio-opaque, un élément radioactif ou des combinaisons de ceux-ci.

9. Formulation de bio-encre selon l'une quelconque des revendications 1 à 8, **caractérisée en outre en ce qu'**elle est stérile.

10. Formulation de bio-encre selon l'une quelconque des revendications 1 à 9, **caractérisée en outre en ce qu'**elle est à une température de 37°C.

11. Utilisation d'une formulation de bio-encre selon l'une quelconque des revendications 1 à 10, dans un processus de bio-impression.

12. Utilisation d'une formulation de bio-encre selon l'une quelconque des revendications 2 à 10, pour la production de matrices comprenant des cellules progénitrices de la lignée myéloïde ou lymphoïde, des cellules souches ou des cellules différenciées de la lignée hématopoïétique.

13. Procédé de bio-impression pour la production d'une matrice 3D, comprenant les étapes suivantes consistant à :
a) bio-imprimer la formulation de bio-encre selon l'une quelconque des revendications 2 à 10 à travers une buse ayant un calibre compris entre 18G et 27G à une pression d'extrusion comprise entre 5 et 200 kPa et à une vitesse d'impression comprise entre 5 et 1 000 mm/min sur un substrat pour obtenir une matrice 3D monocouche ou multicouche ;
b) tremper la matrice 3D obtenue à l'étape a) dans une solution de réticulation ;
c) ajouter le milieu de culture cellulaire à l'échafaudage et éventuellement des facteurs de croissance pour favoriser la différenciation cellulaire.

14. Procédé de bio-impression pour la production d'une matrice 3D selon la revendication 13, dans lequel la pression d'extrusion est comprise entre 5kPa et 20 kPa et la vitesse d'impression est comprise entre 400 et 1 000 mm/min.

15. Matrice pouvant être obtenue au moyen d'un procédé de bio-impression de la formulation de bio-encre selon l'une quelconque des revendications 1 à 10.

16. Matrice pouvant être obtenue au moyen d'un procédé de bio-impression de la formulation de bio-encre selon l'une quelconque des revendications 13 à 14.

17. Matrice selon l'une quelconque des revendications 15 à 16, comprenant en outre une ou plusieurs molécules choisies dans le groupe constitué par :
- les composants de la matrice extracellulaire tels que les protéoglycanes, l'acide hyaluronique, les collagènes, l'élastine, la fibronectine, la fibrine, le fibrinogène, la laminine, la thrombospondine ;
- les facteurs de croissance, les cytokines et les chimiokines, de préférence hématopoïétiques, tels que les interleukines, CSF-1, G-CSF, GM-CSF, SCF, FLT3-L, TPO, EPO, SDF-1α ; TGF-β1, TNFα, VEGF, FGF, les ligands de Notch, WNT, l'angiopoïétine-1, BMP, IGF-2 et leurs fragments ou variantes ;
- les antigènes, les anticorps conjugués ou non, ou leurs fragments tels que les CDR ou les épitopes ;
- les hormones, telles que l'insuline, le glucagon, la triiodothyronine, la thyroxine ou leurs antagonistes ;
- les médicaments ou promédicaments, tels que les mimétiques de la TPO, les agonistes des récepteurs de la TPO, les agonistes des récepteurs de la tyrosine kinase, les inhibiteurs des récepteurs de la tyrosine kinase, les inhibiteurs de la Rho kinase (ROCK), les inhibiteurs de la kinase, les antagonistes des récepteurs des hydrocarbures aryliques, les agents chimiothérapeutiques ;
- les acides nucléiques, tels que l'ADN, l'ARN, le siARN, le ARNi et le microARN, les plasmides, les lentivirus, le CRISPR ;
- les enzymes et/ou les intermédiaires des réactions enzymatiques ;
- les toxines.

18. Procédé de dissolution de la matrice obtenue par un procédé de bio-impression de la formulation de bio-encre selon l'une quelconque des revendications 1 à 10, dans lequel le procédé comprend la préparation de la matrice au moyen du procédé selon la revendication 13 et comprend en outre l'utilisation d'une solution de dissolution de la matrice comprenant du citrate de sodium, de la collagénase, de l'alginate lyase et une source de glucose.

19. Procédé selon la revendication 18, dans lequel la solution de dissolution de la matrice comprend :
- du citrate de sodium 15 mM
- de la collagénase 10 U / mL
- de l'alginate lyase 5 U / mL
- 5 mM de glucose
- une solution tampon saline

20. Utilisation de la matrice selon l'une quelconque des revendications 15 à 17, comme modèle ex vivo pour soutenir l'hématopoïèse ou pour la production de plaquettes et de cellules sanguines.

21. Matrice selon l'une quelconque des revendications 15 à 17, destinée à être utilisée comme implant chirurgical pour favoriser l'hématopoïèse in vivo ou la production de plaquettes et de cellules sanguines.
